## Europäisches Patentamt
## European Patent Office
## Office européen des brevets

(19)

(11) Veröffentlichungsnummer: **0 019 814**
**B1**

(12) **EUROPÄISCHE PATENTSCHRIFT**

(45) Veröffentlichungstag der Patentschrift:
30.05.84

(51) Int. Cl.³: **A 61 M 5/14,** G 06 F 15/42

(21) Anmeldenummer: **80102731.9**

(22) Anmeldetag: **16.05.80**

(54) **Steuervorrichtung für Infusionsgeräte.**

(30) Priorität: **23.05.79 DE 2920976**

(43) Veröffentlichungstag der Anmeldung:
**10.12.80 Patentblatt 80/25**

(45) Bekanntmachung des Hinweises auf die Patenterteilung:
**30.05.84 Patentblatt 84/22**

(84) Benannte Vertragsstaaten:
**AT BE CH DE FR GB IT LI NL SE**

(56) Entgegenhaltungen:
**EP - A - 0 002 775**
**EP - A - 0 002 776**
**GB - A - 2 011 652**

(73) Patentinhaber: **SIEMENS AKTIENGESELLSCHAFT,**
**Berlin und München Wittelsbacherplatz 2,**
**D-8000 München 2 (DE)**

(72) Erfinder: **Franetzki, Manfred, Dr.-Ing., Schleifweg 7 b,**
**D-8521 Uttenreuth (DE)**
Erfinder: **Prestele, Karl, Dipl.-Phys.,**
**Bismarckstrasse 21 d, D-8520 Erlangen (DE)**

**Beschreibung**

Die Erfindung bezieht sich auf eine Steuervorrichtung für Infusionsgeräte, die einerseits aus einem in einem Patientenkörper implantierbaren Gerätegehäuse mit einer Förder- und Dosiereinheit zum dosierbaren Transport der Infusionsflüssigkeit aus einem Vorratsbehälter zur Ausflussöffnung eines Katheters einschliesslich zugehöriger Betriebsschaltung und die andererseits aus einem separaten Steuer- oder Programmiergerät für die Betriebsschaltung bestehen, wobei zwecks induktiver Signalübertragung das Steuer- oder Programmiergerät eine erste Spule und die Betriebsschaltung eine zweite Spule und wobei zwecks Codierung der zu übertragenen Signale das Steuer- oder Programmiergerät einen Codierer und die Betriebsschaltung einen Decodierer aufweisen.

Bei der Behandlung von Patienten mit flüssigen Medikamenten ist eine fest eingestellte Rate der Infusionsflüssigkeit häufig unzureichend, und es soll daher möglich sein, die pro Zeiteinheit infundierte Flüssigkeitsmenge in bestimmten Zeitabständen nachzuregeln, umzusteuern oder auch programmässig zu verändern. Dies ist insbesondere dann erforderlich, wenn zur Diabetes-Therapie Insulin fortwährend infundiert werden soll, weil der Insulinbedarf des Zuckerkranken während des Tages, bedingt z. B. durch den Rhythmus der Mahlzeiten, grossen Schwankungen unterworfen ist, während er hingegen während der Nacht zeitlich nahezu konstant ist.

Aus der DE-B-25 13 467 ist ein Infusionsgerät für vorstehend angegebenen Zweck bekannt, bei der der eigentliche Medikamentengeber mit Förder- und Dosiereinheit als Implantat ausgebildet ist. Dazu sind sämtliche Bauelemente des Medikamentengebers in einem Gehäuse aus gewebeverträglichem Material, das gegebenenfalls mit einem Metallschutz, beispielsweise aus Titan, versehen ist, eingebaut. Der Programmgeber für den Medikamentengeber wird hingegen extrakorporal vom Patienten getragen. Zur Übermittlung der Steuersignale vom Programmgeber zur Betriebsschaltung des Medikamentengebers dient eine magnetische Kopplung mittels Induktionsspulen im Medikamentengeber und Programmgeber. Weiterhin sind aus den älteren, nicht vorveröffentlichten Patentdokumenten EP-A-0002776 und EP-A-0002775 Vorrichtungen zur programmierbaren Infusion von Flüssigkeiten in den menschlichen oder tierischen Körper vorbekannt, welche Steuervorrichtungen der eingangs genannten Art benötigen. In beiden Dokumenten wird davon ausgegangen, dass die Infusionsgeräte alternativ implantierbar oder vom Patienten extrakorporal tragbar ausgebildet sind, wobei aber in jedem Fall jeweils ein separates Steuer- oder Programmiergerät vorhanden sein muss. Mit dem Steuer- oder Programmiergerät kann entweder jeweils die Infusionsrate entsprechend dem sich ändernden Bedarf umgesteuert oder ein Zeitprogramm vorgegeben werden. Die Signalübertragung vom Steuer- oder Programmiergerät zum Infusionsgerät mit Betriebsschaltung soll bei diesem Stand der Technik auch bereits induktiv und codiert erfolgen, wozu entsprechende Spulen und Codierer bzw. Decodierer vorhanden sind. Es werden jedoch keine Aussagen darüber gemacht, wie im einzelnen die Programmübertragung erfolgen soll.

Zur Fernübertragung von Signalen in einen Patientenkörper hinein hat sich die beschriebene induktive Signalübertragung insbesondere deswegen bewährt, da hierfür keine zusätzlichen Antennen ausserhalb des implantierten Gerätes notwendig sind. Die Reichweite solcher magnetischer Kopplung ist allerdings vergleichsweise gering und wird im wesentlichen durch die geometrischen und elektrischen Parameter des magnetischen Senders bestimmt. Nachteilig ist bei einem solchen induktiven Signalübertragungssystem weiterhin, dass auf die Induktionsspule möglicherweise neben dem eingentlichen Senderfeld auch andere magnetische Felder aus der Umgebung einwirken können. Speziell bei Infusionsgeräten besteht dann die Gefahr, dass solche Magnetfelder möglicherweise in der Betriebsschaltung Steuersignale für die Förder- und Dosiereinheit erzeugen können, wodurch erhöhter Flüssigkeitsausstoss mit unter Umständen lebensgefährlichen Folgen bewirkt werden könnte. Aus diesem Grunde werden bei der beschriebenen Steuervorrichtung die Signale codiert übertragen.

Aufgabe der Erfindung ist es daher, die vorbekannte Steuervorrichtung für implantierbare Infusionsgeräte derart weiterzubilden, dass die Signalübertragung unter weitestgehender Ausschaltung aller äusserer Einflussmöglichkeiten erfolgt. Eine ungewollte Aktivierung der Förder- und Dosiereinheit muss mit Sicherheit vermieden werden.

Die Aufgabe wird erfindungsgemäss dadurch gelöst, dass mittels Codierer/Decodierer entweder jedes Steuersignal zur Einstellung einer veränderbaren Infusionsrate der Förder- und Dosiereinheit einzeln mit einem Schlüsselsignal nach vorgegebenem Code oder eine gesamte Signalfolge als Steuerprogramm für zeitlich vorgegebene Infusionsraten mit einem gemeinsamen Schlüsselsignal nach vorgegebenem Code übertragen werden, welche Steuersignale oder Steuerprogramme in Speichern, die der Betriebsschaltung für die Förder- und Dosiereinheit zugeordnet sind, abgelegt werden.

Mit der Erfindung ist also erreicht, dass lediglich mittels eines Schlüsselsignales nach vorgegebenem Code Steuersignale in der Betriebsschaltung im Gerätegehäuse zur Aktivierung der Förder- und Dosiereinheit zwecks Abgabe von Flüssigkeit nach vorgebbarem Programm wirksam werden. Bei der alternativen Steuersignalübertragung ist sichergestellt, dass die Betriebsschaltung für die Förder- und Dosiereinheit im Gerätegehäuse nicht unbeabsichtigt aktiviert werden kann.

Vorzugsweise ist die Induktivität der ersten Spule als Sendespule durch eine darauf abgestimmte Kapazität zu einem Serienresonanzkreis ergänzt, wobei die Reichweite der so erzeugten magnetischen Übertragungsfelder durch die geometrischen und elektrischen Parameter des Resonanzkreises einerseits sowie der zugehörigen Verstärkerstufen im Empfänger andererseits bestimmt werden. Der von der ersten Spule gebildeten Sendespule ist ein Treiber sowie ein Oszillator vorgeschaltet, so dass ein magnetisches Übertragungsfeld mit einer durch den Oszillator bestimmten Trägerfrequenz erzeugt wird. Dementsprechend wird die Induktivität der zweiten Spule in der Betriebsschaltung des implantierten Infusionsgerätes durch eine geeignete Kapazität zu einem Parallelresonanzkreis ergänzt, der auf die Trägerfrequenz des magnetischen Übertragungsfeldes abgestimmt und somit als selektive Antenne für das Senderfeld wirksam wird.

In einer vorteilhaften Ausgestaltung der erfindungsgemässen Steuervorrichtung erzeugt der Codierer im Steuergerät aus jedem Steuerimpuls einen Doppelimpuls mit definiertem Abstand und definierten Pulsbreite, wobei der Decodierer der Betriebsschaltung im Gerätegehäuse nur dann einen Steuerimpuls für die Förder- und Dosiereinheit erzeugt, wenn nach einem empfangenen Doppelimpuls mit definiertem Pulsabstand und definierte Pulsbreite in einem vorbestimmbaren Zeitintervall kein weiterer Impuls folgt. Während bei dieser Ausbildung der Erfindung jeder Steuerimpuls für die Steuerschaltung im Dosiergerät einzeln codiert übertragen wird, ist in anderer Ausbildung der Erfindung auch möglich, ein vollständiges Programm insgesamt codiert zu übertragen.

Bei einer solchen Ausbildung der Erfindung sind der Betriebsschaltung im Infusionsgerät Speicher zugeordnet, in denen das mittels Codierer/Decodierer codiert übertragene Steuerprogramm abgelegt wird. Ein solcher Speicher kann aus zwei Zählern bestehen, wenn im Infusionsgerät lediglich zwei Variable verändert werden und ansonsten nach vorgegebenem Programm gearbeitet wird. Dies ist z. B. dann der Fall, wenn das Infusionsgerät mit einer einstellbaren, zeitlich konstanten Infusionsrate als Basalrate arbeitet, der bei Bedarf eine zusätzliche zeitlich begrenzte Infusionsrate als Abrufprogramm aufgesetzt werden soll. In Weiterbildung der Erfindung kann auch ein solcher Speicher benutzt werden, der eine Vorprogrammierung der Infusionsrate über den Tagesverlauf mit vorbestimmbarer Dosierung ermöglicht.

Schliesslich können in bevorzugter Weiterbildung der Erfindung die der Betriebsschaltung zugeordneten Speicher auch Pufferspeicher mit n Speicherplätzen umfassen, aus dem die Speicherinformation nur in der gleichen zeitlichen Reihenfolge ausgelesen werden kann, wie sie eingelesen wurde. Ein solcher Speicher wird vorzugsweise durch ein sogenanntes FIFO gebildet.

Mit der Erfindung sind also prinzipiell zwei unterschiedliche Steuerprinzipien möglich. Einerseits kann ein externes Steuergerät ständig körpernah getragen werden, wobei dann das Steuergerät ständig die aktive Steuerfunktion für das implantierte Infusionsgerät übernimmt. Im anderen Fall wird ein externes Programmiergerät mit Signalübertrager lediglich zur Umprogrammierung des Speichers an das Infusionsgerät herangeführt. Dabei kann entweder eine Umprogrammierung der konstanten Basalrate der Infusion und Eingabe eines abzuarbeitenden Aufsetzprogramms mit erhöhter Infusionsrate oder auch die Vorprogrammierung eines ganzen Tagesprofils der Infusion mit sich zeitlich ändernden Infusionsraten erfolgen. Speziell bei Verwendung des FIFO's als Pufferspeicher lassen sich die Abrufprogramme mit sich zeitlich ändernden Infusionsraten optimal an den beispielsweise bei Einnahme einer Mahlzeit auftretenden Bedarf anpassen. Gemeinsam ist bei den verschiedenen Ausbildungen der Erfindung, dass die Signalübertragung mittels erfindungsgemässen Codiersystem so sicher erfolgt, dass in der Betriebsschaltung keine ungewollten Steuersignale erzeugt werden und damit Fehlschaltungen ausgeschlossen sind.

Weitere Vorteile und Einzelheiten der Erfindung ergeben sich aus der nachfolgenden Figurenbeschreibung von Ausführungsbeispielen anhand der Zeichnung in Verbindung mit weiteren Unteransprüchen.

Es zeigen

Fig. 1 schematisch einen Patienten mit implantiertem Infusionsgerät und extrakorporal getragenem Steuergerät,

Fig. 2 Aufbau und Funktionsweise der Steuervorrichtung bei einer Geräteanordnung nach Fig. 1,

Fig. 3 schematisch einen Patienten mit implantiertem Infusionsgerät und induktiv angekoppeltem externen Steuergerät bei Umsteuerung der Betriebsschaltung im Infusionsgerät,

Fig. 4 Aufbau der Steuervorrichtung bei einer Geräteanordnung nach Fig. 3,

Fig. 5 Aufbau der Betriebsschaltung nach Fig. 4 zur Umsteuerung von Basalrate und Abrufprogramm,

Fig. 6 schematisch einen Patientenkörper mit implantiertem Infusionsgerät, einem Programmiergerät zur Vorprogrammierung eines Tagesprogramms sowie einer Sendespule,

Fig. 7 die Steuervorrichtung der Geräteanordnung nach Fig. 6 als Blockschaltbild,

Fig. 8 Aufbau der Betriebsschaltung zur Programmierung eines Tagesprogrammes,

Fig. 9 ein Zeitdiagramm der Infusionsrate mit konstanter Basalrate und zwei verschiedenen Abrufprogrammen.

Fig. 10 Aufbau einer Betriebsschaltung mit Pufferspeicher zur Realisierung von Abrufprogrammen mit sich zeitlich ändernder Infusionsrate.

Fig. 11 und 12 zwei verschiedene extrakorporale Steuergeräte der Betriebsschaltung nach Fig. 10.

In den Figuren sind identische Teile mit den gleichen Bezugszeichen versehen.

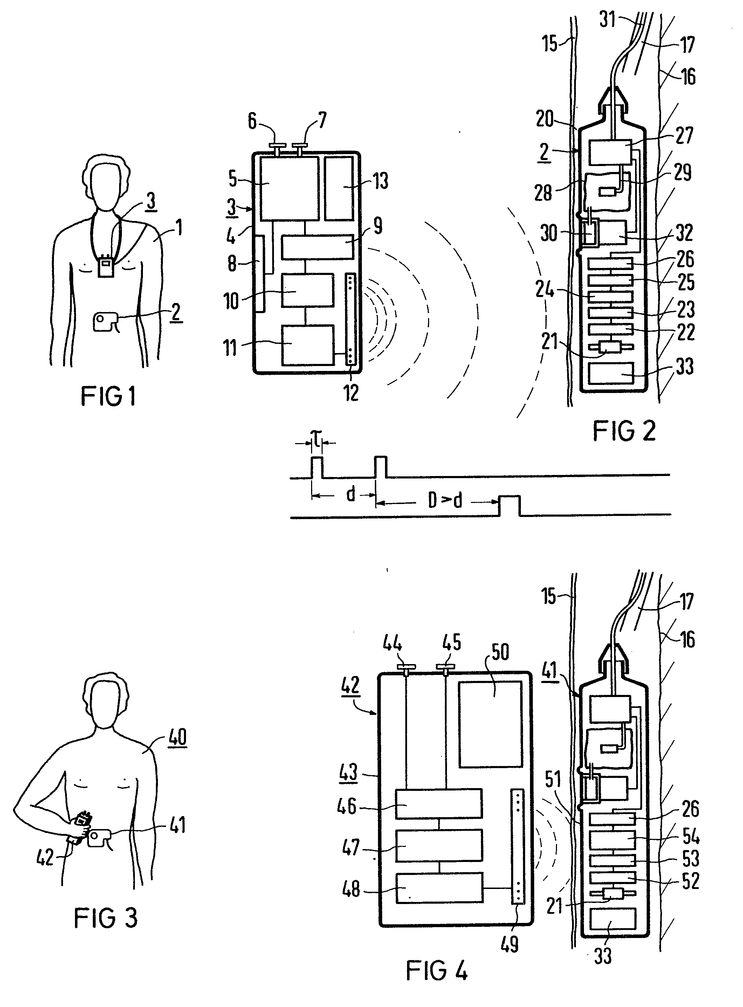

Die Fig. 1 zeigt einen Patienten 1, dem im Bauchbereich ein Infusionsgerät 2 implantiert ist. Das Implantat besteht aus einer flachen Kapsel, die unmittelbar unter der Haut im Muskel bzw. Fettgewebe plaziert ist, und von dem ein Katheter innerhalb des Körpers in eine Vene gelegt ist. Extrakorporal trägt der Patient 1 an einem Halsband oder dgl. ein Steuergerät 3, das induktiv an die Betriebsschaltung im Gerätegehäuse des implantierten Infusionsgerätes angekoppelt ist. Das Steuergerät 3 kann ebensogut in einer Tasche der Körperkleidung getragen werden.

In der Fig. 2 sind Infusionsgerät 2 und Steuergerät 3 in Wirkverbindung dargestellt: Das Steuergerät 3 besteht aus einem Gerätegehäuse 4, das als Programmgeber eine Einheit 5 zur Schaltung von Basalraten und Abrufprogrammen aufweist. Die Einheit 5 ist von aussen über zwei Stellglieder 6 und 7 betätigbar. Die Stellglieder 6 und 7 sind zweckmässigerweise als Stufenschalter mit Wahlscheiben ausgebildet. Mit dem ersten Stellglied 6 kann eine Basalrate der Infusion, z. B. zwischen 0,4 und 2,0 IE/h (Internationale (Isulin)–Einheiten pro Stunde) für die Insulininfusion eingestellt werden, während am Stellglied 7 zusätzliche Dosismengen von einigen IE angewählt werden, die nach festem Programm in einem vorbestimmten Zeitraum, etwa einer Stunde, als erhöhte Infusionsrate abgegeben werden. Vom Programmgeber 5 werden in Serie ein Codierer 9, ein Oszillator 10 und ein Treiber 11 angesteuert, über die eine Sendespule 12 zur Erzeugung eines magnetischen Wechselfeldes gespeist wird. Dem Programmgeber 5 ist eine Anzeigeeinheit 8 aus Flüssigkristallen zur digitalen Anzeige von angewählter Basalrate und/oder Infusionsdosis parallelgeschaltet. Weiterhin ist im Gehäuse 4 eine Batterie 13 als Energiequelle vorhanden.

Das implantierte Infusionsgerät 2 besteht aus einer Gehäusekapsel 20 aus körperverträglichem Material, beispielsweise aus Titan, in ähnlich flacher Bauform, wie sie bisher auch von Herzschrittmachern bekannt war. Das Gerätegehäuse 20 ist im Patientenkörper unmittelbar unter der Gewebehaut 15 oberhalb des Muskel- und Fettgewebes 16 in der Nähe einer Körpervene 17 implantiert. Im Gehäuse 20 befinden sich eine Empfangsspule 21 mit einem in Serie nachgeschalteten Wechselspannungsverstärker 22, einem Impulsverstärker 23, einem Impulsformer 24 und einem Decodierer 25, von dem ein Motortreiber 26 angesteuert wird. Der Motortreiber 26 betätigt den Schrittmotor einer Rollenpumpe 27 als aktive Förder- und Dosiereinheit. Mittels Rollenpumpe 27 wird die Infusionsflüssigkeit aus einem Vorratsbehälter 28 über einen Förderschlauch 29 bis zum Anschluss eines Katheters 31 gefördert. Der Katheter 31 ist mit seinem proximalen Ende in der Vene 17 plaziert, so dass die Infusion intravenös erfolgt. Der Vorratsbehälter 28 ist mit einem Nachfüllstutzen 30 in der Gehäusewand verbunden, die so unter der Haut 15 plaziert ist, dass bei Bedarf mit einer Spritze Infusionsflüssigkeit in den Vorratsbehälter 28 nachgefüllt werden kann. Weiterhin ist im Gehäuse 20 eine Alarmeinheit 32 und eine Batterie 33 zur Energieversorgung des implantierten Infusionsgerätes 2 vorhanden.

Bei der so beschriebenen Ausführungsform der Erfindung wird die Insulinangabe in jedem Moment vom externen Steuergerät 3 gesteuert. Der Sender muss also derart ausgelegt sein, dass sich der Empfänger ständig in Signalreichweite befindet. Aufgrund der starken Abstandsabhängigkeit von magnetischen Feldern ($1/r^3$–Abhängigkeit) muss ein starkes Senderfeld erzeugt sowie die Empfängerschaltung entsprechend empfindlich ausgelegt werden, da eine Reichweite von ca. 0,5 m sichergestellt sein muss.

Die Steuersignalübertragung mittels Codierer/Decodierer verläuft folgendermassen: Der Codierer 9 erzeugt aus jedem Steuerimpuls des Programmgebers einen Doppelimpuls mit definiertem Abstand d und definierter Pulsbreite τ. Der Oszillator 10 liefert eine Trägerfrequenz im Mittelfrequenzbereich zwischen 10 und 30 kHz. Eine solche Trägerfrequenz ist erforderlich, um die Metallkapsel 20 des implantierten Infusionsgerätes 2 zu durchdringen. Der Treiber 11 liefert den Strom für die Sendespule. Ergänzt man die Induktivität der Sendespule 13 durch einen Kondensator zu einem Serienresonanzkreis, so wird die Effektivität der Senderspule um die Güte q des Resonanzkreises erhöht.

Durch Ansteuerung des Oszillators 10 vom Codierer 9 wird die Trägerfrequenz mit den codierten Impulsen getastet. Das von der Sendespule 12 erzeugte magnetische Wechselfeld weist also ein Impulsmuster entsprechend der vorgegebenen Tastung auf. Dieses getastete magnetische Wechselfeld induziert in die Empfangsspule des Gerätegehäuses 20 im Patientenkörper eine Wechselspannung.

Als Empfangsspule 21 wird eine Ferritspule verwendet, deren Induktivität mit der Kapazität eines Kondensators zu einem Parallelresonanzkreis ergänzt ist, welcher auf die Trägerfrequenz des Senderoszillators 10 abgestimmt ist. Die induzierte Wechselspannung wird im Wechselspannungsverstärker 22 und nachfolgendem Impulsverstärker 23 verstärkt. Dabei ist der Impulsverstärker mit seiner unteren Grenzfrequenz auf die Impulsbreite τ der codierten Signale abgestimmt. Vom Impulsformer 24 wird dann dementsprechend jeweils ein Rechteckimpuls erzeugt. Diese Rechteckimpulse gelangen zum Decodierer 25. Der Decodierer 25 erzeugt aus einem zu ihm gelangenden Doppelimpuls mit definiertem Abstand d dann und nur dann einen Steuerimpuls für den Motortreiber 26, wenn nach dem Doppelimpuls in einem vorgegebenen Zeitintervall D > d kein weiterer Impuls folgt. Dadurch werden Störimpulse mit grosser Sicherheit ausgeschaltet. Insbesondere wird damit auch verhindert, dass möglicherweise periodisch anfallende Störimpulse, die zufällig die Frequenz der codierten Signale haben könnten, eine Steuerfunktion auslösen und so eine fehlerhafte Infusionsrate bewirken.

Die Struktur der codierten Signale ist in der Fig. 2 noch zusätzlich dargestellt. Durch die drei Codierparameter τ, d und D ist mit genügender Sicherheit gewährleistet, dass nur Steuersignale des Signalgebers 5 die Pumpe 27 wirksam steuern können.

Die Fig. 3 zeigt einen Patienten 40 mit einem implantierten Infusionsgerät 41. Vom Patienten 40 ist mit der Hand ein Umsteuergerät 42 extrakorporal an den Bauchbereich des implantierten Infusionsgerätes 41 anlegbar, wobei durch die Haut mit dem Steuergerät 42 die in zwei der Betriebsschaltung des Infusionsgerätes 41 zugeordneten Speichern fest eingespeicherte Infusionsrate und Zusatzdosis umgesteuert werden kann.

Das Umsteuergerät 42 in Fig. 4 hat an seinem Gerätegehäuse 43 als Programmgeber lediglich die Stellglieder 44 und 45 zur Anwahl von Basalrate und Abrufdosis, die unmittelbar auf eine Codierelektronikeinheit 46 einwirken. Der Codiereinheit 46 ist entsprechend Fig. 2 wiederum ein Oszillator 47, ein Treiber 48 sowie eine Sendespule 49 nachgeschaltet. Im Gehäuse 43 ist weiterhin eine Batterie 50 als Energieversorgung angeordnet.

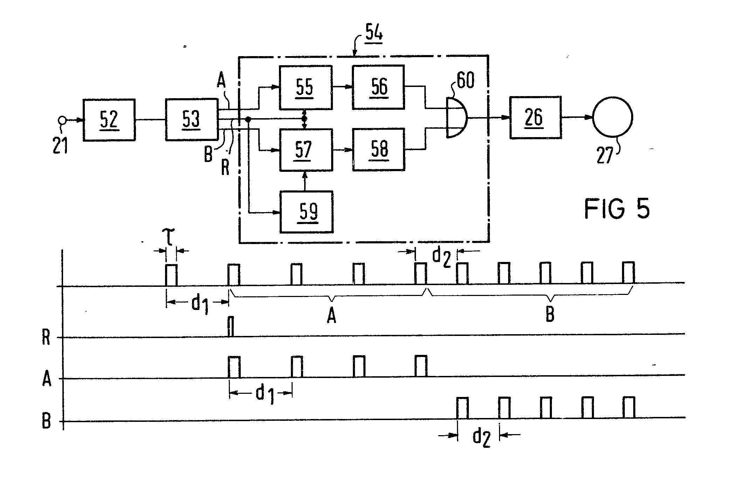

Das zugehörige Infusionsgerät 41 hat neben der anhand Fig. 2 beschriebenen Empfängerspule sowie Einheiten 26 bis 33 eine anders ausgelegte Betriebsschaltung. Von der Empfängerspule 21 wird ein Empfangsverstärker 52 mit nachgeschaltetem Decodierer 53 angesteuert. Dem Decodierer 53 ist eine Speicher- und Steuerschaltung 54 nachgeschaltet, die in Fig. 5 im einzelnen beschrieben wird. Von dieser speziell ausgelegten Speicherschaltung 54 wird in oben beschriebener Weise ein Motortreiber 26 für die Infusionspumpe 27 aktiviert.

Die Signalübertragung erfolgt bei diesem Ausführungsbeispiel nur über einen geringen Abstand von maximal 0,1m, da nur bei unmittelbarem Auflegen des Steuergerätes die Betriebsschaltung des implantierten Infusionsgrätes umgesteuert werden soll.

In der Fig. 5 bedeutet 21, 26, 27, 52 und 53 wiederum die aus Fig. 4 bekannten Einheiten. Die Speicher- und Steuerschaltung 54 besteht im wesentlichen aus zwei Zählern 55 und 57 mit jeweils nachgeschalteten Taktgebern 56 und 58 sowie einem Ein-Stunden-Zeitgeber 59 und einem logischen Verknüpfungsglied 60. Dabei hat die Decodiereinheit 53 drei Ausgänge A, B und R. Vom Ausgang A wird ein erster Zähler 55 als Basalratenspeicher mit Taktgeber 56 angesteuert; vom zweiten Ausgang B ein zweiter Zähler 57 als Abrufratenspeicher für die Zusatzdosis mit Taktgeber 58. Die Signale der beiden Taktgeber 56 und 58 sind über ein Gatter 60 verknüpft, so dass sich an diesem Ausgang eine Summation der beiden Taktimpulse als Überlagerung von Basalrate und zusätzlicher Abrufrate ergibt. Von diesem Taktsignal wird der Motortreiber 26 angesteuert. Vom Signal-Ausgang R des Decodierers 53 werden vor Umprogrammierung beide Zähler 55 und 57 jeweils auf Null zurückgesetzt. Gleichzeitig triggert das Signal R den Zeitgeber 59, der nach Ablauf einer vorgegebenen Zeit, z. B. 1 h, Zähler 57 auf Null zurücksetzt. Damit ist die zusätzliche Abrufrate abgeschaltet.

Mit dem externen Steuergerät kann also die Betriebsschaltung im Infusionsgerät zu einem beliebigen Zeitpunkt umgesteuert werden. Dies ist im Impulsdiagramm dargestellt: Der Decodierer 53 trennt Impulssalven mit unterschiedlichen Impulsabständen $d_1$ und $d_2$. Entsprechend Fig. 2 erkennt der Decodierer 53 ein erstes Doppelimpulssignal mit Impulsabstand $d_1$ als Schlüsselsignal

und setzt einen Resetimpuls R für die Zähler 55 und 57. Die vorher gespeicherte Information wird also gelöscht. Die erste Impulssalve A mit Impulsabstand $d_1$ lädt dann den ersten Speicher auf den am Steuergerät eingestellten Wert für die Basalrate; die zweite Impulssalve B mit Impulsabstand $d_2$ lädt den zweiten Speicher auf den am Steuergerät eingestellten Wert für die Zusatzdosis. Nach Umsteuerung wird also vom Infusionsgerät eine neu eingestellte Basalrate abgegeben, auf die vom Zeitgeber 59 gesteuert eine eintsprechend eingestellte Abrufdosis als zeitlich erhöhte Rate aufgesetzt ist. Nach Abgabe dieser Abrufdosis erfolgt die Förderung wieder mit eingestellter Basalrate.

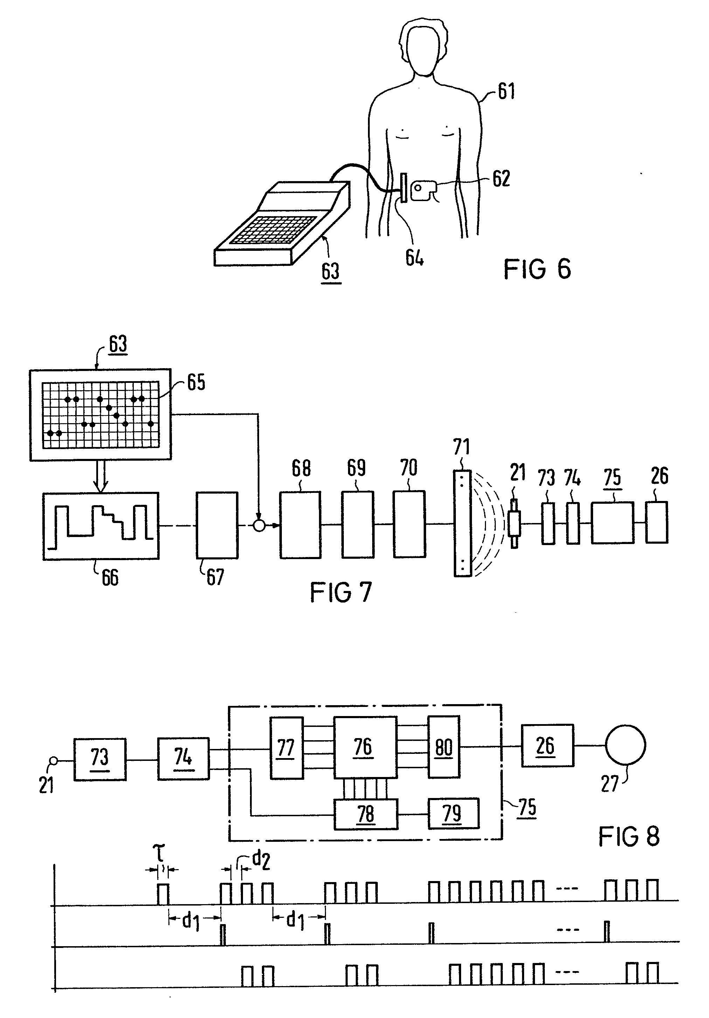

In der Fig. 6 kennzeichnet 61 einen Patienten mit einem implantierten Infusionsgerät 62. Ein externes Programmiergerät 63 ist mit einer Sendespule 64 mittels Kabel oder dgl. verbunden. Das Programmiergerät 63 ist etwa pultförmig ausgebildet und weist ein Raster als Programmierfeld auf, mit dem der Tagesverlauf der Flüssigkeitsabgabe mit sich entsprechend dem Tagesbedarf ändernden Profil vorprogrammiert werden kann. Ein solches Programm wird mit an das implantierte Infusionsgerät anlegbarer Sendespule übertragen.

In der Fig. 7 kennzeichnen 63 das Programmiergerät aus Fig. 6 speziell mit einem Kreuzschienenverteiler 65 als Programmierfeld. Es bedeuten die Zeilen die verschiedene Infusionsraten und die Spalten Zeitintervalle eines beliebig wiederholbaren 24-Stunden-Tages-Rhytmus mit Zeitintervallen von einer oder einer halben Stunde zwischen 0 und 24 Uhr. Am Kreuzschienenverteiler 65 ist also mittels Kontaktstecker ein Tagesprofil programmierbar. Von diesem programmierten Tagesprofil kann bei Bedarf das Profil auf einem Programmträger 66 zwischengespeichert werden. Solche Programmträger sind beispielsweise Lochkarten, Magnetkarten oder ähnliches, die von einem entsprechenden Lesegerät 67 auslesbar sind.

Vom Programmiergerät 63 oder Programmlesegerät 67 wird eine Codierschaltung 68 angesteuert. Die Codierung erfolgt zweckmässigerweise so, dass die einem bestimmten Zeitintervall zugeordnete Infusionsrate in eine Impulssalve mit Impulsabstand $d_2$ gewandelt wird, wobei die Anzahl der Impulse der Rate entspricht. Die Impulssalven der verschiedenen Zeitintervalle werden durch definierte Impulspausen mit Abstand $d_1$ getrennt. Ein erster Doppelimpuls mit Impulsabstand $d_1$ kann als Schlüsselsignal verwendet werden. Am Programmiergerät 63 ist natürlich die aktuelle Tageszeit einstellbar, an der das Einlesen des Programms begonnen wird. Dadurch wird die den Tagesablauf entsprechende zeitsynchrone Programmübertragung gewährleistet.

Der Codierer 68 aktiviert wiederum über einen Oszillator 69 und Treiber 70 eine Sendespule 71. Von der Empfängerspule 21 gelangt das Signal über einen Empfangsverstärker 73 mit Impulsformer auf die Decodiereinheit 74 mit nachgeschalteter Speicher- und Steuerschaltung 75, von der – wie beschrieben – der Motortreiber 26 des Infusionsgerätes aktiviert wird.

Die Speicher- und Steuerschaltung 75 ist in Fig. 8 als Block gestrichelt angedeutet. Bekannt aus obiger Beschreibung sind wiederum die Einheiten 21, 26 und 27 sowie 73 und 74. Wesentlicher Bestandteil der Speicher- und Steuerschaltung 75 ist ein digitaler Halbleiterspeicher 76 (RAM), dem ein Wortzähler 77, Adressenzähler 78, Zeitgeber 79 und Taktgeber 80 zugeordnet sind. Von der Decodiereinheit 73 werden während des Programmiervorgangs jeweils Wort-Impulse und Adressimpulse ausgegeben. Es werden die einzelnen Impulse einer Impulssalve als Wort-Impulse an den Wortzähler 77 weitergegeben, während nach einer Impulspause die Information des Wortzählers 77 in den entsprechenden Speicherplatz eingelegt wird, der Wortzähler 77 zurückgesetzt und der Adressenzähler 78 weitergeschaltet wird.

In den digitalen Halbleiterspeicher 76 kann beispielsweise ein Tagesprogramm der Infusion mit Halbstundenraster und zehn unterschiedlichen Infusionsraten abgelegt werden. Die einzelnen Speicherwerte werden vom Zeitgeber 79 über den Adresszähler 78 tageszeitsynchron abgerufen und an den digital steuerbaren Taktgeber 80 angelegt, dessen Ausgangssignale den Motortreiber 26 ansteuern.

Das Ausführungsbeispiel des Infusionsgerätes nach Fig. 6 bis 8 wird vorteilhaft im klinischen Bereich eingesetzt, wo vom Arzt am stationären Programmiergerät das Tagesprogramm der Infusion entsprechend den individuellen Bedürfnissen des Patienten eingestellt werden soll. Es ist jedoch auch möglich, dass vom Patienten selbst auf Programmträgern gespeicherte Tagesprogramme mit einem mobilen Übertragungsgerät eingelesen werden. Ein solches Gerät entspricht im wesentlichen dem Steuergerät 42 nach Fig. 3, wobei statt der Stellglieder für Basal- und Abrufrate eine Programmleseinheit benötigt wird. Vom Patienten können dann beispielsweise aus einer Programmbibliothek für einen Arbeitstag, einen Ruhetag oder dergleichen entsprechende Programme auf Lochkarten oder Magnetkarten entnommen werden und ein Übertragungsgerät mit Kartenleser 67 zum Auslesen des Programmes eingeschoben werden, wobei mit Auslesen unmittelbar die Übertragung und Einlesen in den digitalen Speicher 76 des implantierten Gerätes erfolgt.

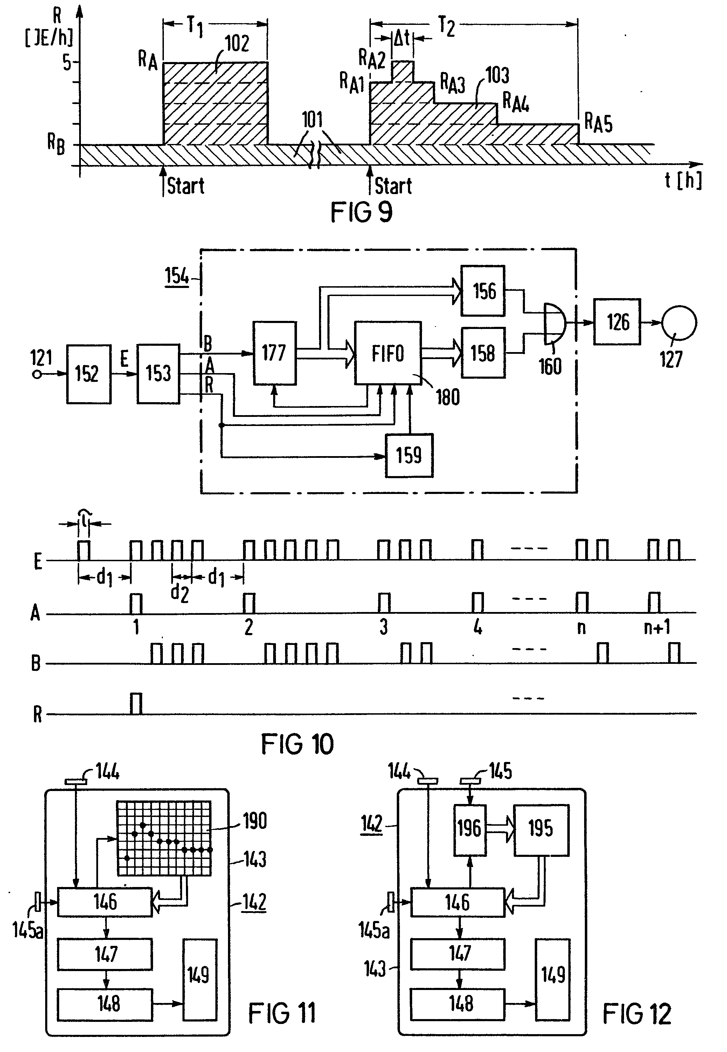

In der Fig. 9 ist die Infusionsrate als Zeitfunktion dargestellt. Im Diagramm bedeuten 101 eine konstante zeitlich nicht begrenzte, aber einstellbare Basalrate $R_B$ der Infusion, die beispielsweise 1 IE/h betragen kann. Auf die Basalrate $R_B$ sind zwei zusätzliche Infusionsdosismengen mit erhöhter Infusionsrate $R_{Ai}$ ausgesetzt. Die strichlierte Fläche 102 und 103 entspricht jeweils der gesamten Zusatzdosis, welche im Ausführungsbeispiel in beiden Fällen gleich gewählt wurden. Beim vorher beschriebenen Ausführungsbeispiel gemäss Fig. 4 konnte mit den vorhandenen Speichern das Abrufprogramm 102 lediglich bezüglich seiner Höhe (Infusionsrate) verändert werden. Gemäss Position 103 lässt sich nun aus Elementarrechtecken aufgebaut ein Profil des Aufsetzprogrammes nachbilden, das den physiologischen Gegebenheiten optimal angepasst ist.

In der Fig. 10 kennzeichnen 121 einen Signalempfänger, 152 einen Empfangsverstärker und 153 einen Decodierer, die entsprechend der vorangehenden Ausführungsbeispiele hintereinandergeschaltet sind und eine Betriebsschaltung 154 ansteuern. Von der Betriebsschaltung 154 wird ein Motortreiber 126 angesteuert, der wiederum einen Schrittmotor 127 der Förder- und Dosiereinheit aktiviert: Soweit entsprechen die beschriebenen Einheiten denen der Fig. 4.

Die Betriebsschaltung 154 besteht aus zwei Taktgebern 156 und 158, einem Zeitgeber 159 und einem logischen Verknüpfungsglied 160 (ODER-Gatter) zur ODER-Verknüpfung der Signale der beiden Taktgeber 156 und 158. Vom Decodierer 153 wird jetzt ein Zähler 177 angesteuert, der einerseits den ersten Taktgeber 156 ansteuert und andererseits während des Programmiervorgangs als Wortzähler für in ein sog. FIFO 180 einzulesende Information wirksam wird. Vom Ausgang des FIFO 180 wird der zweite Taktgeber 158 angesteuert. Decodierer 153, FIFO 180 und Wortzähler 177 sind ausserdem über Steuerleistungen miteinander verbunden. Ein FIFO (first in – first out) ist ein digitaler Pufferspeicher mit n Speicherplätzen, einem mehr-bit-Dateneingang, einem mehr-bit-Datenausgsng sowie zugehörigen Steuerleitungen. Diese Pufferspeicher geben die gespeicherte Information in gleicher Reihenfolge aus, in der sie eingelesen wurde, und benötigt keine separate Adressierlogik. FIFO's sind also für den vorliegenden Zweck in idealer Weise geeignet.

Zur Programmierung der Betriebsschaltung 154 auf ein Abrufprogramm gemäss Fig. 9, rechte Seite, wird die Signalfolge erfindungsgemäss mit Schlüsselsignalen versehen und vom externen Steuer- oder Programmiergerät mittels Sendespule kodiert auf die Empfangsspule im Infusionsgerät übertragen. Die Impulsfolge mit Impulsbreiten $\tau$ und Impulspausen $d_1$ zur Definition eines Doppelimpulses als schlüsselsignal sowie mit weiteren Impulspausen $d_2$ werden vom Decodierer 153 erkannt. Im Impulsdiagramm ist in der Zeile E die am Decodierer anstehende Impulsfolge dargestellt. Vom Ausgang A wird jeweils bei einem Doppelimpuls ein Signal auf das FIFO 180 und damit ein Befehl, im Speicherregister einen Schritt weiter zu gehen, gegeben. Diese Impulse sind in der Zeile A mit 1 bis n + 1 gekennzeichnet. Nach Erkennen eines Schlüsselsignals werden im Decodierer die darauf folgenden Pulse mit Abstand $d_2$ am Ausgang B zur Verfügung gestellt; die Summe einer Impulssalve entspricht jeweils der zu speichernden Infusionsrate und ist auf der Zeile B dargestellt. Diese wird vom Wortzähler in eine entsprechende Binärzahl umgewandelt und jeweils bei Vorliegen eines Signals am Ausgang A (nach Doppelimpuls mit Abstand $d_1$) in das FIFO 180 eingelesen. Sobald die Information vom FIFO 180 übernommen ist, wird von diesem an den Wortzähler 177 ein Resetimpuls abgegeben. Anschliessend wird durch Aufaddieren der nächsten Impulssalve eine neue Speicherinformation gebildet und über den Wortzähler anschliessend in das FIFO 180 eingeschoben.

Der Wortzähler 177 hat eine Doppelfunktion in der Betriebsschaltung. Er dient einerseits beim Programmieren als Wortzähler zum Einlesen des Abrufratenprofils; andererseits wird er auch als Speicher für die Basalrate verwendet, weshalb seine Ausgänge auch mit dem steuerbaren Basalratentaktgeber 156 verbunden sind. Die zuletzt anstehende Impulssalve vom Decodierer stellt die Basalrateninformation dar. Da anschliessend kein Impuls zum Weitersetzen des FIFO's gegeben wird, wird diese Information nicht in den Speicher eingelesen; sie wird auch nicht gelöscht, sondern bleibt bis zum nächsten Programmiervorgang als Basalrate erhalten.

Beim ersten Doppelimpuls mit Abstand $d_1$ wird zusätzlich ein Reset-Impuls zur Rücksetzung des FIFO's 180 und des Zeitgebers 159 erzeugt. Dadurch ist einerseits der Zeitnullpunkt für das Abrufprogramm festgelegt und andererseits verhindert, dass die beim letzten Programmiervorgang gespeicherte Basalrateninformation in das FIFO 180 eingelesen wird.

Zur Abarbeitung des Abrufprogramms wird vom Zeitgeber 159 ein Zeittakt erzeugt, der beispielsweise 10 Minuten betragen kann. Bei Verwendung eines handelsüblichen FIFO's mit 16 Speicherplätzen à 4 bit kann dann ein derartiges Abrufprogramm insgesamt eine Zeitspanne von 160 Minuten umfassen. Das Abrufratenprofil lässt sich in 16 Schritten auch in der Höhe der Infusionsraten gut an die jeweiligen Bedürfnisse anpassen.

In den Figuren 11 und 12 bedeuten jeweils 142 das externe Steuer- bzw. Programmiergerät. Wie bereits beschrieben ist dabei im Gehäuse 143 eine Codierstufe 146 mit nachfolgendem Treiber 147 und Oszillator 148 angeordnet, von denen eine Senderspule 149 angesteuert wird. Zur Einstellung der Basalrate ist ein Schalter 144 vorhanden.

Im Gegensatz zum Ausführungsbeispiel nach Fig. 4 wird hier durch den Abrufprogrammgeber keine feste Zusatzrate eingestellt, sondern ein Abrufprogramm mit sich ändernden Infusionsraten. In der Fig. 11 ist zur manuellen Programmierung ein Kreuzschienenverteiler 190 vorhanden, der (z. B. in 10 Minuten-Intervallen) ein Zeitraster bildet, denen entsprechende Infusionsraten zugeordnet werden können. Über eine Starttaste 145a wird nach manueller Programmierung des Abrufprogrammes die erfindungsgemässe Signalübertragung gestartet und damit das Infusionsgerät im Sinne der programmässig vorgegebenen Abgabe der zusätzlichen Infusionsdosis gesteuert.

Statt des manuell programmierbaren Kreuzschienenverteilers können als Programmgeber auch Lochkarten- bzw. Lochstreifenleser oder Magnetkartenleser angeordnet sein. Bei solchen Ausführungsbeispielen werden dem Patienten auf entsprechende Medien gespeicherte Programme zur Verfügung gestellt, die über den Leser in die Codiereinheit und Programmübertragungseinheit eingegeben werden.

In ganz entsprechender Ausbildung eines Programmiergerätes wird gemäss Fig. 12 ein fest installierter Halbleiterspeicher verwendet. Dazu eignen sich vorzugsweise sog. PROM's (Programmable Read Only Memory). Ein solcher Halbleiterspeicher ist mit 195 gekennzeichnet, wobei dem PROM 195 ein Adressenregister 196 zugeordnet ist, das über den Schalter 145 angesteuert wird. Das PROM 195 kann m x n Speicherworte aufnehmen. Das heisst also, es können m komplette Abrufprogramme einprogrammiert werden, die beispielsweise für einen Patienten individuell optimiert sind. Über das Adressenregister 196 ist der Zugriff zu diesen m Einzelprogrammen gegeben. Vom Patienten braucht lediglich am Abrufprogrammwahlschalter 145 das entsprechende Programm angewählt und die Starttaste 145a betätigt zu werden. Die Programmierung des Infusionsgerätes im Sinne der Abgabe eines Abrufprogrammes läuft dann selbsttätig ab. Während des Programmiervorganges wird das Adressenregister 196 jeweils von der Codierstufe solange weitergeschaltet, bis die n Speicherinformationen des angewählten Programmes abgearbeitet sind. Anschliessend wird die eingestellte Basalrate codiert und ebenfalls in die Betriebsschaltung übertragen.

In einer anderen Ausführungsform kann der Abrufprogrammgeber auch aus zwei Stufenschaltern bestehen, von denen der eine Zeitintervallen und der andere den Infusionsraten bzw. Dosismengen zugeordnet ist. Es können damit am Steuer- bzw. Programmiergerät beispielsweise eine Abrufdosis und die zeitliche Dauer eines Abrufprofils eingestellt werden.

Mit den verschiedenen Ausführungsbeispielen der Erfindung steht nun für die Praxis eine Generation von implantierbaren Geräten zur Verfügung, die für die unterschiedlichsten Anforderungen im klinischen und ausserklinischen Bereich zugeschnitten sind. Durch Realisierung des erfindungsgemässen Steuer- bzw. Programmübertragungsprinzips ist dabei immer die notwendige Sicherheit gewährleistet.

**Patentansprüche**

1. Steuervorrichtung für Infusionsgeräte, die einerseits aus einem in einem Patientenkörper (1, 40, 61) implantierbaren Gerätegehäuse (2, 41, 62) mit einer Förder- und Dosiereinheit (27) zum dosierbaren Transport der Infusionsflüssigkeit aus einem Vorratsbehälter (28) zur Ausflussöffnung eines Katheters (31) einschliesslich zugehöriger Betriebsschaltung und die andererseits aus einem separaten Steuer- oder Programmiergerät (3, 42, 63, 142) für die Betriebsschaltung bestehen, wobei zwecks induktiver Signalübertragung das Steuer- oder Programmiergerät (3, 42, 63, 142) eine erste Spule (12, 49, 64, 71, 149) und die Betriebsschaltung eine zweite Spule (21, 121) und wobei zwecks Codierung der zu übertragenden Signale das Steuer- oder Programmiergerät einen Codierer (9, 46, 68, 146) und die Betriebsschaltung einen Decodierer (25, 53, 74, 153) aufweisen, dadurch gekennzeichnet, dass mittels Codierer/Decodierer (9/25, 46/53, 68/74, 146/153) entweder jedes Steuersignal zur Einstellung einer veränderbaren Infusionsrate der Förder- und Dosiereinheit (27) einzeln mit einem Schlüsselsignal nach vorgegebenem Code oder eine gesamte Signalfolge als Steuerprogramm für zeitlich vorgegebene Infusionsraten mit einem gemeinsamen Schlüsselsignal nach vorgegebenem Code übertragen werden, welche Steuerprogramme in Speichern (55, 57, 76, 180), die der Betriebsschaltung für die Förder- und Dosiereinheit (27) zugeordnet sind, abgelegt werden.

2. Steuervorrichtung nach Anspruch 1, dadurch gekennzeichnet, dass die erste Spule (12, 49, 71) im Steuer- oder Programmiergerät (3, 42, 63) durch eine entsprechende ihrer Induktivität abgestimmte Kapazität zu einem Serienresonanzkreis ergänzt ist.

3. Steuervorrichtung nach Anspruch 1, dadurch gekennzeichnet, dass der ersten Spule (12, 49, 71) als Sendespule ein Treiber (11) vorgeschaltet ist, so dass das magnetische Senderfeld durch die geometrischen Parameter der ersten Spule (12, 49, 71) und durch den Speisestrom des Treibers bestimmt ist.

4. Steuervorrichtung nach Anspruch 3, dadurch gekennzeichnet, dass dem der Senderspule vorgeschalteten Treiber (11, 48) ein Oszillator (10, 47) zugeordnet ist.

5. Steuervorrichtung nach Anspruch 1, dadurch gekennzeichnet, dass die zweite Spule (21) der Betriebsschaltung eine Ferritspule ist, die durch eine entsprechend ihrer Induktivität abgestimmte Kapazität zu einem Parallelresonanzkreis ergänzt ist, dessen Resonanzfrequenz auf die Frequenz des Oszillators (10, 47) im Steuer- oder Programmiergerät (3, 42, 63) abgestimmt ist.

6. Steuervorrichtung nach Anspruch 1 oder 4, dadurch gekennzeichnet, dass in der Betriebsschaltung zwischen zweiter Spule (21) und Decodierer (25) ein Wechselspannungsverstärker (22), ein Impulsverstärker (23) sowie eine Impulsformerstufe (24) angeordnet sind.

7. Steuervorrichtung nach Anspruch 1, dadurch gekennzeichnet, dass der Codierer (9, 46) im Steuer- oder Programmiergerät (3, 42) aus jedem Eingabeimpuls einen Doppelimpuls mit definiertem Abstand (d) und definierter Impulsbreite ($\tau$) erzeugt und dass der Decodierer (25, 53, 74) der Betriebsschaltung nur dann einen Steuerimpuls für Förder- und Dosiereinheit (27) erzeugt, wenn nach einem Doppelimpuls mit definiertem Pulsabstand (d) und definierter Pulsbreite ($\tau$) in einem vorbestimmbaren Zeitintervall (D > d) kein weiteres Signal erfolgt.

8. Steuervorrichtung nach Anspruch 1, dadurch gekennzeichnet, dass die Speicher einzelne Zähler (55, 57) umfassen.

9. Steuervorrichtung nach Anspruch 8, dadurch gekennzeichnet, dass durch den Zählerstand eines ersten Zählers (55) eine zeitlich konstante Infusionsrate der Förder- und Dosiereinheit (27) als Basalrate festgelegt ist und dass durch den Zählerstand eines zweiten Zählers (57) eine zusätzliche Infusionsrate der Förder- und Dosiereinheit (27) für ein von einem Zeitgeber vorgegebenes Zeitintervall hinzugefügt wird.

10. Steuervorrichtung nach Anspruch 1, dadurch gekennzeichnet, dass die Betriebsschaltung im implantierbaren Gerätegehäuse (62) einen digitalen Speicher (76) als Programmspeicher enthält, der ein Tagesprogramm der Infusion entsprechend einem 24-Stunden-Rhytmus aufnehmen kann, welches mittels eines zugeordneten Zeitgebers (79) zyklisch abgearbeitet werden kann.

11. Steuervorrichtung nach Anspruch 10, dadurch gekennzeichnet, dass vom Codierer (68) einzelne bestimmten Zeitintervallen zugeordnete Infusionsraten der Förder- und Dosiereinheit in Impulsgruppen mit definiertem Impulsabstand ($d_2$) gewandelt werden und dass Impulsgruppen verschiedener Zeitintervalle durch Übertragungspausen definierten Abstands ($d_1$) getrennt sind.

12. Steuervorrichtung nach Anspruch 10, dadurch gekennzeichnet, dass dem digitalen Speicher (76) ein Wortzähler (77) und ein Adressenzähler (78) zugeordnet sind und dass von den Übertragungssignalen einerseits der Adressenzähler (78), mit dem die Speicherplätze des Speichers (76) entsprechend dem 24-Stunden-Rhythmus bestimmt sind, und andererseits der Wortzähler (77), mit dem die Infusionsrate bestimmt ist, angesteuert werden.

13. Steuervorrichtung nach Anspruch 10, dadurch gekennzeichnet, dass bei der Übertragung der von vorprogrammierten Infusionsraten entsprechenden Impulsgruppen die Impulspausen definierten Abstandes ($d_1$) zwischen den Impulsgruppen das Einlesen eines der Anzahl der Einzelimpulse entsprechenden Speicherwertes bewirken sowie gleichzeitig den Wortzähler (77) zurück- und den Adressenzähler (78) weitersetzen.

14. Steuervorrichtung nach Anspruch 1, dadurch gekennzeichnet, dass die der Betriebsschaltung zugeordneten Speicher einen Pufferspeicher (180) mit n Speicherplätzen umfassen, aus dem die Speicherinformation nur in der gleichen zeitlichen Reihenfolge ausgelesen werden kann, in der sie eingelesen wurde.

15. Steuervorrichtung nach Anspruch 14, dadurch gekennzeichnet, dass der Pufferspeicher (180) durch ein sog. FIFO (first in – first out) gebildet ist, das einen mehr-bit-Dateneingang, einen mehr-bit-Datenausgang sowie zugehörige Steuereingänge aufweist.

16. Steuervorrichtung nach Anspruch 1, dadurch gekennzeichnet, dass das FIFO (180) eine Speicherkapazität von 16 × 4 bit hat.

17. Steuervorrichtung nach Anspruch 15, dadurch gekennzeichnet, dass dem Dateneingang des FIFO (180) ein Wortzähler (177) zur Umwandlung einer Impulssalve in eine Binärzahl vorgeschaltet ist.

18. Steuervorrichtung nach Anspruch 15, dadurch gekennzeichnet, dass dem FIFO (180) ein Zeitgeber (159) zugeordnet ist.

19. Steuervorrichtung nach Anspruch 1, dadurch gekennzeichnet, dass das separate Steuer-oder Programmiergerät (142) einen Abrufprogrammgeber (180, 190, 195) mit zugehörigem Abrufprogrammwahlschalter enthält.

20. Steuervorrichtung nach Anspruch 19, dadurch gekennzeichnet, dass der Abrufprogrammgeber ein Kreuzschienenverteiler (180) ist.

21. Steuervorrichtung nach Anspruch 19, dadurch gekennzeichnet, dass der Abrufprogrammgeber ein PROM (195) mit m vorgegebenen Abrufprogrammen ist, wobei vom Patienten jeweils eines der vorgegebenen Programme über den Abrufprogrammwahlschalter (145) und das zugehörige Adressregister (196) angewählt werden kann.

22. Steuervorrichtung nach Anspruch 19, dadurch gekennzeichnet, dass der Abrufprogrammgeber ein Magnet- bzw. Lochkartenleser ist.

**Claims**

1. A control device for infusion apparatus which on the one hand consists of a housing (2, 41, 62) implanted in a patient's body (1, 40, 61) and has a delivery and dosage unit (27) for the measured transportation of infusion liquid from a feed container (28) to the outlet of a catheter (31) together with the assigned operating circuit, and which on the other hand consist of a separate control or programming apparatus (3, 42, 63, 142) for the operating circuit, where for inductive signal transmission the control or programming apparatus (3, 42, 63, 142) possesses a first coil (12, 49, 64, 71, 149) and the operating circuit possesses a second coil (21, 121), and where coding of the signals to be transmitted is provided for in the control or programming apparatus by a coder (9, 46, 68, 146) and by a decoder (25, 53, 74, 153) in the operating circuit, characterised in that by means of the coder/decoder (9/25, 46/53, 68/74, 146/153) either each control signal for adjusting a variable infusion rate of the delivery and dosage unit (27) is individually transmitted with a code signal in accordance with a predetermined code or an entire signal sequence as a control programme for timed infusion rates is transmitted with a common code signal in accordance with a predetermined code, which control programmes are held in storage means (55, 57, 76, 180) assigned to the operating circuit for the delivery and dosage unit (27).

2. A control device as claimed in Claim 1, characterised in that first coil (12, 49, 71) in the control or programming apparatus (3, 42, 63) is supplemented by a capacitance matched to its inductance to form a series resonant circuit.

3. A control device as claimed in Claim 1, characterised in that first coil (12, 49, 71) is a transmitting coil preceded by a driver (11) so that the magnetic transmitting field is determined by the geometric parameters of the first coil (12, 49, 71) and by the feed current of the driver.

4. A control device as climed in Claim 3, characterised in that the driver (11, 48) connected preceding the transmitting coil is assigned an oscillator (10, 47).

5. A control device as claimed in Claim 1, characterised in that second coil (21) of the operating circuit is a ferrite coil supplemented by a capacitance matched to its inductance to form a parallel resonant circuit whose resonance frequency is tuned to the frequency of the oscillator (10, 47) in the control or programming apparatus (3, 42, 63).

6. A control device as claimed in Claim 1 or 4, characterised in that between the second coil (21) and the decoder (25) in the operating circuit there are arranged an alternating voltage amplifier (22), a pulse amplifier (23) and a pulse forming stage (24).

7. A control device as claimed in Claim 1, characterised in that the coder (9, 46) in the control or programming device (3, 42) produces a double pulse having a defined spacing (d) and a defined pulse width ($\tau$) from each input pulse, and that the decoder (25, 53, 74) of the operating circuit only produced a control pulse for the delivery and dosage unit (27) if no further signal occurs in a predeterminable time interval (D > d) following a double pulse having a defined pulse spacing (d) and a defined pulse width ($\tau$).

8. A control device as claimed in Claim 1, characterised in that the storage means comprise individual counters (55, 57).

9. A control device as claimed in Claim, characterised in that an infusion rate of the delivery and dosage unit (27) which is constant in terms of time, is determined as a base rate by the count of a first counter (55), and that by the count of a second counter (57) an additional infusion rate of the delivery and dosage unit (27) is added for a time interval given by a timer.

10. A control device as claimed in Claim 1, characterised in that the operating circuit in the implantable housing (62) comprises a digital memory (76) as programme storage means, which in accordance with a 24-hour rhythm can accommodate a daily infusion programme that can be cyclically run through by means of an assigned timer (79).

11. A control device as claimed in Claim 10, characterised in that individual infusion rates of the delivery and dosage unit assigned particular time intervals are converted into pulse groups of a defined pulse spacing ($d_2$) by the coder (68), and that pulse groups of different time intervals are separated by transmission pauses of a defined spacing ($d_1$).

12. A control device as claimed in Claim 10, characterised in that the digital memory (76) is assigned a word counter (77) and an address counter (78), and that the transmission signals operate on the one hand the address counter (78) by means of which the storage places of the memory (76) are determined in accordance with the 24-hour rhythm, and on the other hand the word counter (77) by means of which the infusion rate is determined.

13. A control device as claimed in Claim 10, characterised in that in the transmission of pulse groups corresponding to pre-programmed infusion rates the pulse pauses of defined spacing ($d_1$) between the pulse groups effect the read-in process of a storage value corresponding to the number of the individual pulses and simultaneously reset the word counter (77) and advance the address counter (78).

14. A control device as claimed in Claim 1, characterised in that the storage means assigned to the operating circuit comprise a buffer memory (180) which has n storage places and from which the storage information can only be read out in the same time sequence in which it was read in.

15. A control device as claimed in Claim 14, characterised in that buffer memory (18) is formed by a so-called FIFO (first in – first out) which has an extra bit data input, an extra bit data output and assigned control inputs.

16. A control device as claimed in Claim 1, characterised in that the FIFO (180) has a storage capacity of 16 × 4 bits.

17. A control device as claimed in Claim 15, characterised in that in order to convert a pulse group into a binary number a word counter (17) is connected preceding the data input of the FIFO (180).

18. A control device as claimed in Claim 15, characterised in that the FIFO (180) is assigned a timer (159).

19. A control device as claimed in Claim 1, characterised in that the separate control or programming apparatus (142) includes a calling programme (180, 190, 195) having an assigned calling programme selection switch.

20. A control device as claimed in Claim 19, characterised in that the calling programme generator is a crossbar distribution panel (180).

21. A control device as claimed in Claim 19, characterised in that the calling programme generator is a PROM (195) having m given calling programmes, whereby the patient can dial one of the predetermined programmes by means of the calling programme selection switch (146) and the assinged address register (196).

22. A control device as claimed in Claim 10, characterised in that the calling programme generator is a magnetic or card reader, as the case may be.

**Revendications**

1. Dispositif de commande pour des appareils d'infusion qui sont constitués d'une part par un boîtier d'appareil (2, 41, 62) implantable dans le corps d'un patient (1, 40, 61) et comportant une unité d'acheminement et de dosage pour le transport dosable du liquide infusé à partir d'un conteneur de réserve (28) vers l'ouverture de sortie d'un cathéter (31), y compris un circuit de fonctionnement associé, et d'autre part par un appareil distinct de commande ou de programmation (3, 42, 63, 142) pour le circuit de fonctionnement, l'appareil de commende ou de programmation (3, 42, 63, 142) comportant une première bobine (12, 49, 64, 71, 149) et le circuit de fonctionnement une seconde bobine (21, 121) pour une transmission inductive des signaux, et l'appareil de commande ou de programmation comportant un codeur (9, 46, 68, 146) et le circuit de fonctionnement in décodeur (25, 53, 74, 153) pour le codage des signaux à transmettre, caractérisé par le fait qu'au moyen du codeur/décodeur (9/25, 46/53, 68/74, 146/153) est transmis soit chaque signal de commande pour le réglage d'un débit d'infusion variable de l'unité d'acheminement et de dosage (27) individuellement avec un signal clé suivant un code prédéterminé, soit une série complète de signaux en tant que programme de commande pour des débits d'infusion prédéterminés dans le temps avec un signal clé commun suivant un code prédéterminé, programmes de commande qui sont mémorisés dans des mémoires (55, 57, 76, 180) qui sont associées au circuit de fonctionnement pour l'unité d'acheminement et de dosage (27).

2. Dispositif de commande suivant la revendication 1, caractérisé par le fait que la première bobine (12, 49, 71) dans l'appareil de commande ou de programmation (3, 42, 63) est complétée par une capacité accordée en correspondance avec son inductance, pour former un circuit résonant série.

3. Dispositif de commande suivant la revendication, caractérisé par le fait qu'en amont de la première bobine (12, 49, 71) servant de bobine émettrice est monté un circuit d'attaque (11) de manière que le champ magnétique émetteur soit déterminé par les paramètres géométriques de la première bobine (12, 49, 71) et par le courant d'alimentation du circuit d'attaque.

4. Diapositif de commande suivant la revendication 3, caractérisé par le fait qu'au circuit d'attaque (11, 48) monté en amont de la bobine émettrice est associé un oscillateur (10, 47).

5. Dispositif de commande suivant la revendication 1, caractérisé par le fait que la seconde bobine (21) du circuit de fonctionnement est une bobine à ferrite qui est complétée par une capacité àccordée en correspondance avec son inductance pour former un circuit résonant parallèle dont la fréquence de résonance est accordée sur la fréquence de l'oscillateur (10, 47) dans l'appareil de commande ou de programmation (3, 42, 63).

6. Dispositif de commande suivant la revendication 1 ou 4, caractérisé par le fait qu'un amplificateur de tension alternative (22), un amplificateur d'impulsions (23) ainsi qu'un étage de mise en forme d'impulsions (24) sont disposés dans le circuit de fonctionnement entre la seconde bobine (21) et le décodeur (25).

7. Dispositif de commande suivant la revendication 1, caractérisé par le fait que le codeur (9, 46) dans l'appareil de commande ou de programmation (3, 42) produit, à partir de chaque impulsion qui lui est appliquée, une impulsion double avec un intervalle défini (d) entre impulsions et une durée d'impulsions définie ($\tau$), et que le décodeur (25, 53, 74) du circuit de fonctionnement ne produit alors une impulsion de commande pour l'unité d'acheminement et de dosage (27) que lorsqu'aucun autre signal n'est produit durant un intervalle de temps (D > d), qui peut être prédéterminé, après une impulsion double avec un intervalle (d)défini entre impulsions et une durée d'impulsions définie ($\tau$).

8. Dispositif de commande suivant la revendication 1, caractérisé par le fait que les mémoires comprennent des compteurs individuels (55, 57).

9. Dispositif de commande suivant la revendication 8, caractérisé par le fait qu'un débit d'infusion constant dans le temps de l'unité d'acheminement et de dosage (27) est déterminé en tant que débit basal par l'état de comptage d'un premier compteur (55), et qu'un débit d'infusion supplémentaire de l'unité d'acheminement et de dosage (27) pour un intervalle de temps prédéterminé par un minuteur est ajouté par l'état de comptage d'un second compteur (57).

10. Dispositif de commande suivant la revendication 1, caractérisé par le fait que le circuit de fonctionnement dans le boîtier d'appareil implantable (62) contient une mémoire numérique (76) en tant que mémoire de programme, qui peut recevoir un programme journalier de l'infusion en correspondance avec un rythme de 24 heures, qui peut être traité cycliquement au moyen d'un minuteur associé (79).

11. Dispositif de commande suivant la revendication 10, caractérisé par le fait que des débits d'infusion individuels associés à des intervalles de temps déterminés sont transformés par le codeur (68) en groupes d'impulsions présentant un intervalle ($d_2$) défini entre impulsions, et que des groupes d'impulsions d'intervalles de temps différents sont séparés par des pauses de transmission correspondant à un intervalle défini ($d_1$).

12. Dispositif de commande suivant la revendication 10, caractérisé par le fait qu'un compteur de mots (77) et un compteur d'adresses (78) sont associés à la mémoire numérique (76), et que d'une part le compteur d'adresses (78), à l'aide duquel les emplacements de mémoire de la mémoire (76) sont déterminés en correspondance avec le rythme de 24 heures, et d'autre part le compteur de mots (77) à l'aide duquel le débit d'infusion est déterminé, sont commandés par les signaux de transmission.

13. Dispositif de commande suivant la revendication 10, caractérisé par le fait que lors de la transmission de groupes d'impulsions correspondant à des débits d'infusion préprogrammés, les pauses entre impulsions correspondant à un intervalle défini ($d_1$) entre les groupes d'impulsions provoquent la mémorisation d'une valeur correspondant au nombre des impulsions individuelles, et simultanément remettent le compteur de mots (77) à zéro et font progresser le compteur d'adresses (78).

14. Dispositif de commande suivant la revendication 1, caractérisé par le fait que les mémoires associées au circuit de fonctionnement comportent une mémoire intermédiaire (180), possédant n emplacements de mémoire, à partir de laquelle l'information mémorisée ne peut être lue que suivant la même séquence temporelle que celle où elle a été mémorisée.

15. Dispositif de commande suivant la revendication 14, caractérisé par le fait que la mémoire intermédiaire (180) est formée par une mémoire dite FIFO (premier entré — premier sorti) qui comporte une entrée de données à plusieurs bits, une sortie de données à plusieurs bits ainsi que des entrées de commande associées.

16. Dispositif de commande suivant la revendication 1, caractérisé par le fait que la mémoire FIFO (180) possède une capacité de mémorisation de 16 × 4 bits.

17. Dispositif de commande suivant la revendication 15, caractérisé par le fait qu'un compteur de mots (177) est monté en amont de l'entrée de données de la mémoire FIFO (180) pour la conversion d'une rafale d'impulsions en un nombre binaire.

18. Dispositif de commande suivant la revendication 15, caractérisé par le fait qu'un minuteur (159) est associé à la mémoire FIFO (180).

19. Dispositif de commande suivant la revendication 1, caractérisé par le fait que l'appareil de commande ou de programmation distinct (142) comporte un générateur de programme d'appel (180, 190, 195), avec un commutateur de sélection de programme d'appel associé.

20. Dispositif de commande suivant la revendication 19, caractérisé par le fait que le générateur de programme d'appel est un répartiteur à barres croisées (180).

21. Dispositif de commande suivant la revendication 19, caractérisé par le fait que le générateur de programme d'appel est une mémoire PROM (195) avec m programmes d'appel prédéterminés, un des programmes prédéterminés pouvant être respectivement sélectionné par le patient par l'intermédiaire du commutateur de sélection de programme d'appel (145) et du registre d'adresses associé (196).

22. Dispositif de commande suivant la revendication 19, caractérisé par le fait que le générateur de programme d'appel est un lecteur de cartes magnétiques ou de cartes perforées.

FIG 1

FIG 2

FIG 3

FIG 4

FIG 5

FIG 6

FIG 7

FIG 8

FIG 9

FIG 10

FIG 11

FIG 12